(19) Europäisches Patentamt
European Patent Office
Office européen des brevets

(11) Numéro de publication: **0 299 866 B1**

(12) **FASCICULE DE BREVET EUROPEEN**

(45) Date de publication de fascicule du brevet: **15.04.92**

(51) Int. Cl.[5]: **C07C 15/16**, C07C 2/86

(21) Numéro de dépôt: **88401813.6**

(22) Date de dépôt: **12.07.88**

(54) **Compositions d'oligomères de polyarylalcanes, leur procédé de fabrication.**

(30) Priorité: **16.07.87 FR 8710067**
**08.10.87 FR 8713912**

(43) Date de publication de la demande:
**18.01.89 Bulletin 89/03**

(45) Mention de la délivrance du brevet:
**15.04.92 Bulletin 92/16**

(84) Etats contractants désignés:
**AT BE CH DE ES FR GB GR IT LI LU NL SE**

(56) Documents cités:
**EP-A- 0 136 230**

(73) Titulaire: **ATOCHEM**
**4 & 8, Cours Michelet La Défense 10**
**F-92800 Puteaux(FR)**

(72) Inventeur: **Commandeur, Raymond**
**Le Rocher Avenue de Venaria**
**F-38220 Vizille(FR)**
Inventeur: **Gurtner, Bernard**
**5 Boulevard Agutte Sambat**
**F-38000 Grenoble(FR)**

**EP 0 299 866 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouvelles compositions d'oligomères de polyarylalcanes et leur procédé de fabrication.

Ces produits sont utiles en tant que solvant de microencapsulation pour papier transfert sans carbone.

L'homme de l'art a déjà proposé dans le brevet EP 136.230 des mélanges d'oligomères sur base de benzyltoluène, dibenzyltoluène et de ditolylphenylméthane qui présentent l'avantage, par rapport à ces mêmes oligomères pris séparément, de cristalliser à très basse température et d'avoir une viscosité toujours compatible avec leur application comme diélectrique pour condensateurs. Ces caractéristiques les rend tout particulièrement aptes à une utilisation en tant que solvant de microencapsulation pour papier transfert sans carbone ; ils possèdent néammoins une caractéristique rédhibitoire pour un tel emploi, à savoir une odeur fortement désagréable qui se révèle tout comme la couleur au moment de la frappe sur machine à écrire, par exemple. Les compositions selon l'invention présentent non seulement une viscosité compatible avec l'application, mais se caractérisent de plus par une "absence d'odeur" absolument indispensable dans l'application visée.

Le brevet GB 1.346.364 résume les propriétés que doivent concilier les solvants de microencapsulation :

- solubiliser le colorant
- ne pas se vaporiser à la mise en oeuvre des microcapsules
- être inerte vis à vis du matériau d'encapsulation
- ne pas réagir avec le révélateur du solvant
- avoir une viscosité faible et peu sensible à la température
- ne pas avoir d'odeur désagréable.

Les inconvénients des mono et dibenzylalkylbenzène étant aussi connus de longue date, de nombreux mélanges de remplacement ont été proposés à partir d'oligomères de polyarylalcanes. Il est cependant toujours difficile de concilier la qualité des propriétés des produits obtenus avec l'aspect économique des moyens d'obtention de ce produit de remplacement.

C'est ainsi que dans la demande de brevet japonais JP-KOKAI 73-86 612 est décrit l'emploi de di-(propylbenzyl)propylbenzène dont la synthèse fait appel à un nombre important d'étapes réactionnelles et de séparation dues à la non-sélectivité de ce type de synthèse.

Enfin, dans la demande de brevet japonais JP-KOKAI 78-42 909 est cité l'emploi en tant que solvant de microencapsulation des isomères suivants :

- ditolylphénylméthane
- tolyldiphénylméthane
- tritolylphénylméthane

La synthèse de chacun de ces isomères nécessite des réactions spécifiques par exemple le tolyldiphénylméthane à partir de chlorure de benzylidène, de toluène et de benzène suivi d'une séparation des isomères, de plus il faut procéder ensuite à un mélange de ces produits, ce qui n'est guère intéressant sur le plan économique.

De façon générale, l'art antérieur montre des produits pour l'application solvant de microencapsulation, du type monobenzylalkylbenzène ou dibenzylalkylbenzène, ou encoure di(alkylphényl)phénylméthane mais qui ne peuvent être fabriqués que par des synthèses peu sélectives.

La présente invention propose des produits adaptés pour l'application solvant de microencapsulation et qui présentent l'avantage de pouvoir être fabriqués de façon simple.

La présente invention concerne des compositions d'oligomères de polyarylalcanes constituées du mélange de deux oligomères A et B caractérisés en ce que :

- l'oligomère A est un mélange d'isomères de formule :

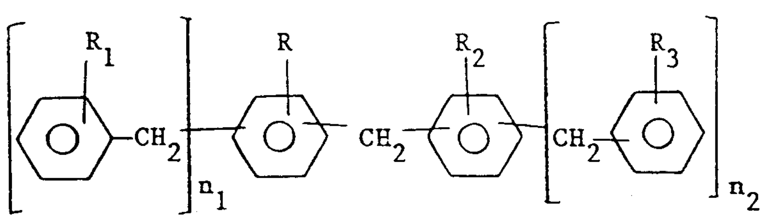

dans laquelle :

- R est une chaîne hydrocarbonée linéaire ou ramifiée à n atomes de carbone, n est compris entre 2 et 16
- $R_1$, $R_2$ et $R_3$ sont identiques ou différents et représentent H ou $CH_3$
- $n_1$ et $n_2$ = 0, 1 et 2 avec $n_1 + n_2 \leq 3$
- chacun des isomères A pouvant avoir des substituants R, $R_1$, $R_2$ et $R_3$ différents :

\- l'oligomère B est un mélange d'isomères de formule :

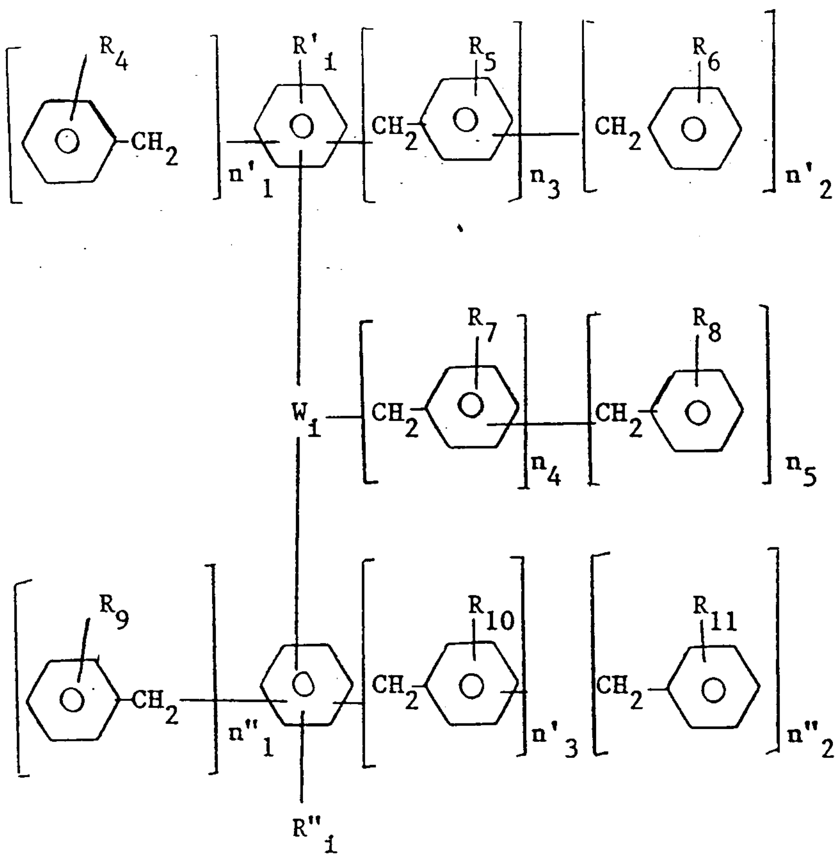

dans laquelle :

- $R'_i$ et $R''_i$ sont identiques ou différents et ont la même signification que R,
- $R_4$, $R_5$, $R_6$, $R_7$, $R_8$, $R_9$, $R_{10}$, $R_{11}$ sont identiques ou différents et représentent H ou $CH_3$,
- $n'_1$, $n''_1$ et $n_4$ = 0, 1 ou 2,
- $n'_2$, $n''_2$, $n_3$, $n'_3$ et $n_5$ = 0 ou 1,
- $n'_1 + n''_1 + n'_2 + n''_2 + n_3 + n'_3 + n_4 + n_5 \leq 2$,
- i vaut 1 ou 2,
- $W_i$ est un groupe de liaison trivalent tel que :

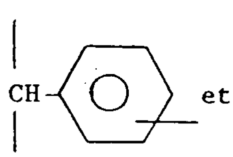

et/ou

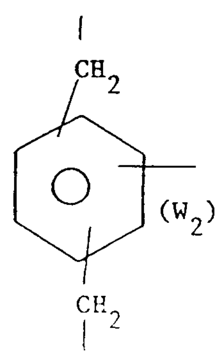

($W_1$)

($W_2$)

et dont les liaisons vers les groupes

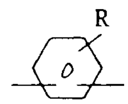

sont assurées par des atomes de carbone n'appartenant pas au groupement phényle de $W_i$.

- chacun des isomères B pouvant avoir des substituants $R'_i$, $R''_i$, $R_4$ à $R_{11}$ différents

C'est-à-dire que B peut être un mélange des produits de formule (I) et (II), chacun des produits (I) et (II) étant lui-même un mélange d'isomères.

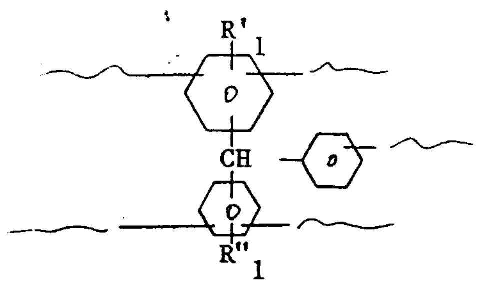

(I)

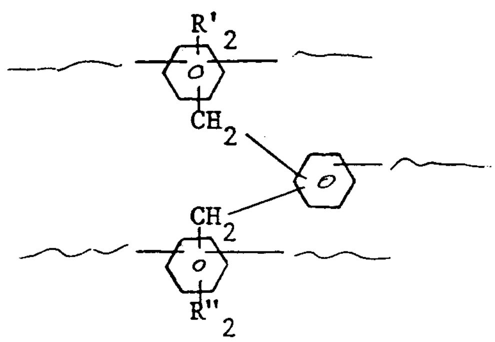

(II)

Parmi les compositions de l'invention on utilise avantageusement celles ou R, $R'_i$ et $R''_i$ sont des alkyles avec n compris entre 3 et 8, et de préférence des isopropyles.

On utilise aussi avantageusement les compositions dans lesquelles $R_1$ à $R_{11}$ sont des atomes d'hydrogène.

On utilise aussi avantageusement les compositions dans lesquelles $R_1$ à $R_{11}$ sont des atomes d'hydrogène et R, $R'_i$ et $R''_i$ sont des alkyles avec n compris entre 3 et 8, et de préférence des groupes isopropyles.

Les compositions particulièrement préférées sont telles que :

- $R_1$ à $R_{11}$ sont des hydrogènes
- R, $R'_i$ et $R''_i$ sont des isopropyles
- i vaut 1, c'est-à-dire que le groupe de liaison $W_i$ est :

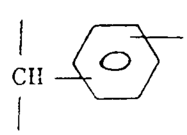

Les compositions selon l'invention présentent toutes les propriétés que doivent concilier les solvants de microencapsulation et la demanderesse a constaté avec surprise que ces produits ne sont pas odorants.

Les compositions selon l'invention peuvent être obtenues en mettant en oeuvre un procédé ne faisant appel en tant que matières premières qu'au chlore, au toluène, au xylène et au produit de formule (III)

(III) 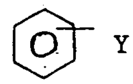

dans laquelle Y est une chaîne hydrocarbonée linéaire ou ramifiée ayant de 2 à 16 atomes de carbone. Le produit de formule (III) peut être aussi un mélange de produits ayant des chaînes Y differentes.

Un procédé d'obtention des compositions d'oligomères selon l'invention est caractérisé en ce que dans une première étape, on fait réagir le chlore sur le toluène ou le xylène ou un mélange de toluène et de xylène par réaction radicalaire en présence de générateur de radicaux, puis on élimine le toluène ou le xylène non réagi, et dans une seconde étape on soumet le produit de réaction de cette première étape à l'action d'un halogénure minéral ou d'un acide minéral en présence d'un produit de formule (III) ou d'un mélange de produits (III) ayant des chaînes Y différentes.

Dans la première étape l'hydrocarbure de départ est soit du toluène, soit du xylène, soit un mélange de toluène et de xylène.

La chloration radicalaire de l'hydrocarbure est habituellement réalisée à une température comprise entre 50 et 110 ° C et mieux entre 70 et 100 ° C. Elle est de préférence menée de telle sorte qu'on ne transforme que 10 à 30 %, exprimé en pourcentage molaire, de l'hydrocarbure engagé en dérivé chloré correspondant. L'hydrocarbure non transformé est ensuite éliminé, par exemple par distillation. En tant que générateur de radicaux libres, on peut employer soit une initiation photochimique, soit un initiateur chimique ; parmi les initiateurs chimiques, on peut citer les composés azo comme l'azodiisobutyronitrile ou encore l'azodivaléronitrile, les péroxydes, comme par exemple le péroxyde de lauroyle. La quantité d'initiateur chimique mise en oeuvre est généralement comprise entre 0,05 et 3 % en poids par rapport à l'hydrocarbure engagé, et de préférence entre 0,1 et 1,5 %.

Le milieu réactionnel obtenu durant la première étape est ensuite en présence du produit de formule (III) soumis à l'action d'un halogénure minéral, ou encore d'un acide minéral. Cette réaction a lieu en pratique à une température comprise entre 30 et 140 ° C, et de préférence entre 50 et 120 ° C.

On peut utiliser un seul produit de formule (III) ou un mélange de produits (III) ayant des chaînes Y différentes.

Avantageusement on ajoute le produit (III) ou ses mélanges dans le milieu obtenu en fin de première étape après l'élimination du toluène et/ou du xylène.

De préférence on ajoute autant de moles de produit (III) que de toluène et/ou de xylène éliminé.

Parmi les halogénures minéraux, on peut utiliser le chlorure ferrique, le trichlorure d'antimoine, le tétrachlorure de titane ou encore le chlorure d'aluminium à des teneurs pondérales par rapport au milieu réactionnel compris habituellement entre 50 ppm et 1 % et de préférence entre 100 ppm et 0,5 %. Les acides minéraux peuvent également être utilisés : l'acide sulfurique par exemple à une concentration pondérale comprise entre 70 et 95 %. Il est aussi possible d'employer les zeolithes ou encore certains oxydes minéraux. Une variante du procédé au niveau de cette deuxième étape consiste à couler le mélange réactionnel de la première étape dans le produit (III) ou le produit (III) et le mélange d'oligomères selon l'invention, contenant l'halogénure ou l'acide minéral sous forme de solution ou de dispersion ; cette variante est particulièrement intéressante pour la mise en continu d'un tel procédé car il est bien entendu que cette synthèse est réalisable en discontinu et en continu.

On peut, après distillation du produit (III) en excès, procéder à l'élimination de l'halogénure minéral ou de l'acide minéral par toute technique connue telle que : lavage à l'eau, neutralisation, séchage.

On utilise avantageusement dans la première étape le toluène, les groupes $R_1$ à $R_{11}$ (dans les oligomères selon l'invention) sont alors des atomes d'hydrogène, et Wi est

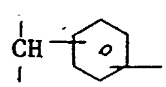

Avantageusement le produit de formule (III) est un alkylbenzène, Y ayant de 3 à 8 atomes de carbone, R, $R'_i$ et $R''_i$ sont alors des alkyles. On utilise de préférence de l'isopropylbenzène (ou cumère), c'est-à-dire que Y est un groupe isopropyle, les groupes R, $R'_i$ et $R''_i$ sont alors identiques et représentent un isopropyle.

Selon le procédé décrit on obtient, de façon courante, directement le mélange d'oligomères de polyarylalcanes dans les proportions pondérales suivantes :

Composé A en mélange d'isomères :

$n_1 + n_2 = 0$ compris entre 56 et 90 %

$n_1 + n_2 = 1$ compris entre 7 et 28 %
$n_1 + n_2 = 2$ compris entre 1,5 et 8 %
$n_1 + n_2 = 3$ compris entre 0,1 et 3 %

Composé B en mélange d'isomères :

$n'_1 = n''_1 + n'_2 + n''_2 + n_3 + n'_3 + n_4 + n_5 = 0$ compris entre 1,1 et 5 %
$n'_1 + n''_1 + n'_2 + n''_2 + n_3 + n'_3 + n_4 + n_5 = 1$ compris entre 0,25 et 1,5 %
$n'_1 + n''_1 + n'_2 + n''_2 + n_3 + n'_3 + n_4 + n_5 = 2$ compris entre 0,05 et 0,5 %

Suivant l'usage applicatif du mélange d'oligomères de polyarylalcanes selon l'invention, il peut être intéressant de procéder à une évaporation-flash de ce mélange pour éliminer les traces d'impuretés provenant soit des matières premières, soit du procédé, ou ayant une origine accidentelle ; dans tous les cas, leurs teneurs pondérales ne dépassent pas 1 à 2 %. Parmi les appareils utilisables, on préfèrera un évaporateur à film mince ; il faut signaler cependant qu'au niveau industriel, les possibilités techniques de tels appareils au niveau de la tenue au vide ne permettent pas toujours de récupérer l'intégralité du mélange d'oligomères de polyarylalcanes : ces produits évaporés font néanmoins partie intégrante de l'invention, comme c'est le cas en particulier des isomères du composé A pour $n_1 + n_2 = 3$ et du composé B pour $n'_1 + n''_1 + n'_2 + n''_2 + n_3 + n'_3 + n_4 + n_5 = 2$.

Les exemples suivants illustrent l'invention sans la limiter.

Exemple 1

Dans un réacteur muni d'une agitation d'un condenseur, d'un tube d'alimentation de chlore et d'une lampe PHILIPS TLADK de 30 Watt, on place 368 g de toluène (4 moles) ; on introduit ensuite 71 g de chlore gazeux (1 mole) en maintenant la température à 80 °C durant 1 heure.

Après arrêt de l'initiation photochimique, le milieu est placé dans un ballon tricol de 1 litre surmonté d'une colonne adiabatique en verre remplie par un garnissage de ressorts de verre (environ 10 plateaux). Le mélange est soumis à une distillation fractionnée sous un vide de 50 mm de mercure de manière à séparer le toluène non réagi. La distillation est arrêtée lorsque la teneur en toluène dans le pied de la colonne est inférieure à 0,2 % (température égale à 100 °C).

Le produit récupéré en pied de distillation est placé dans une ampoule de coulage et il est introduit en une heure dans un réacteur muni d'une agitation contenant 600 g de cumène (5 moles) et 60 mg de $FeCl_3$ d'une température de 100 °C. L'ensemble est maintenu encore 1 heure à 100 °C sous agitation après fin de coulage.

Le milieu réactionnel, après refroidissement, est lavé à l'acide chlorydrique 10 %, puis à l'eau jusqu'à neutralité et enfin le cumène en excès est éliminé par distillation sous vide de 10 mn de mercure avec une colonne de quelques plateaux de manière à ce que la teneur résiduelle en cumène dans le produit en pied soit inférieure à 500 ppm (température de pied en fin de distillation = 180 °C).

Le mélange d'oligomères de polyarylalcanes obtenu a la composition pondérale suivante :

| PRODUIT | $n_1 + n_2$ | | | | $n'_1 + n''_1 + n'_2 + n''_2 + n_3 + n'_3 + n_4 + n_5$ | | |
|---|---|---|---|---|---|---|---|
| | 0 | 1 | 2 | 3 | 0 | 1 | 2 |
| A | 68,8 | 20,8 | 4,2 | 2,5 | - | - | - |
| B | - | - | - | - | 2,4 | 1 | 0,3 |

Et sa formule est telle que :
$R = R'_i = R''_i = CH(CH_3)_2$
$R_1$ à $R_{11} = H$
$i = 1$, c'est-à-dire $W_1$ est

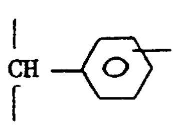

Le rendement pondéral calculé par rapport au cumène réagi est de 97 %.

Ce produit soumis à une évaporation flash à 300 °C sous 1 mm de Hg, donne avec un rendement de

95 % un mélange d'oligomères de polyarylalcanes très faiblement odorant, dont la composition diffère avec celle donnée ci-dessus par l'absence des produits correspondants à :

A et $n_1 + n_2 = 3$

B et $n'_1 + n''_1 + n'_2 + n''_2 + n_3 + n'_3 + n_4 + n_5 = 2$

Pour une viscosité :

à 20 °C = 9,5 cps

à 40 °C = 4,40 cps

EXEMPLE 2

En opérant dans les mêmes conditions de l'exemple 1, mais en utilisant 6 moles de toluène (592 g) à la photochloration pour 1 mole de chlore et 9 moles de cumère dans la réaction de couplage, on obtient un mélange d'oligomères de polyarylalcanes de même formule que dans l'exemple 1 et ayant la composition pondérale suivante :

| PRODUIT | $n_1 + n_2$ | | | | $n'_1 + n''_1 + n'_2 + n''_2 + n_3 + n'_3 + n_4 + n_5$ | | |
|---|---|---|---|---|---|---|---|
| | 0 | 1 | 2 | 3 | 0 | 1 | 2 |
| A | 83 % | 11 % | 2,8 % | 0,7% | - | - | - |
| B | - | - | - | - | 2 % | 0,4 % | 0,1 % |

Le rendement pondéral calculé par rapport au cumère est de 98 %.

Ce produit soumis à une évaporation flash à 300 °C sous 1 mm de Hg donne avec un rendement de 98 % un mélange d'oligomères de polyarylalcanes très faiblement odorant dont la composition diffère avec celle donnée ci-dessus par l'absence des produits correspondants à :

A et $n_1 + n_2 = 3$

B et $n'_1 + n''_1 + n'_2 + n''_2 + n_3 + n'_3 + n_4 + n_5 = 2$

Pour une viscosité à :

20 °C = 6,9 cps

40 °C = 3,9 cps

EXEMPLE 3

En opérant dans les mêmes conditions que l'exemple 1 mais en remplaçant le cumène par 5 moles d'éthylbenzène (530 g) à la réaction de couplage.

Le mélange d'oligomères de polyarylalcanes obtenu a la composition pondérale suivante :

| PRODUIT | $n_1 + n_2$ | | | | $n'_1 + n''_1 + n'_2 + n''_2 + n_3 + n'_3 + n_4 + n_5$ | | |
|---|---|---|---|---|---|---|---|
| | 0 | 1 | 2 | 3 | 0 | 1 | 2 |
| A | 67 | 22,3 | 4,4 | 2,6 | - | - | - |
| B | - | - | - | - | 2,4 | 0,9 | 0,4 |

Le rendement pondéral calculé par rapport à l'éthylbenzène est de 98 %.

Et une formule dans laquelle :

$R, R'_i, R''_i = C_2H_5$

$R_1$ à $R_{11}$ = H

i = 1

$W_i = W_1 =$ 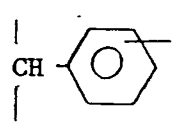

Ce produit soumis à une évaporation flash à 300 °C sous 1 mm de Hg donne avec un rendement de 96

%, un mélange d'oligomères de polyarylalcanes faiblement odorant dont la composition diffère avec celle donnée ci-dessus par l'absence des produits correspondants à :

A et $n_1 + n_2 = 3$

B et $n'_1 + n''_1 + n'_2 + n''_2 + n_3 + n'_3 + n_4 + n_5 = 2$

Pour une viscosité à :

20°C = 6,9 cps

40°C = 4,2 cps

## Revendications

**1.** Compositions d'oligomères de polyarylalcanes constituées du mélange de deux oligomères A et B caractérisés en ce que :

- l'oligomère A est un mélange d'isomères de formule :

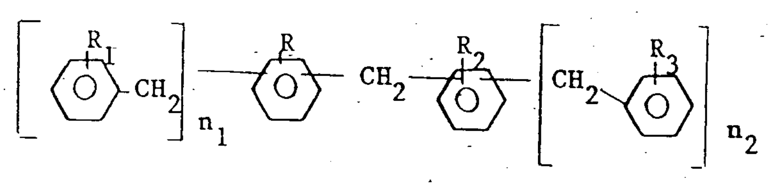

dans laquelle :

- R est une chaîne hydrocarbonée linéaire ou ramifiée à n atomes de carbone, n est compris entre 2 et 16
- $R_1$, $R_2$ et $R_3$ sont identiques ou différents et représentent H ou $CH_3$
- $n_1$ et $n_2$ = 0, 1 et 2 avec $n_1 + n_2 \leq 3$
- chacun des isomères A pouvant avoir des substituants R, $R_1$, $R_2$ et $R_3$ différents :

- l'oligomère B est un mélange d'isomères de formule :

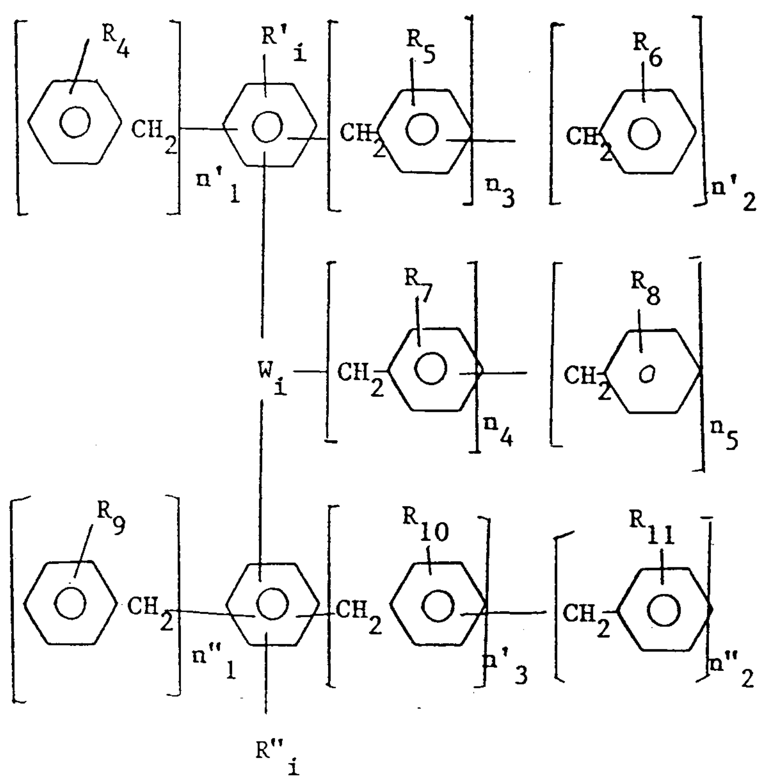

dans laquelle :

- $R'_i$ et $R''_i$ sont identiques ou différents et ont la même signification que R,

- $R_4$, $R_5$, $R_6$, $R_7$, $R_8$, $R_9$, $R_{10}$, $R_{11}$ sont identiques ou différents et représentent H ou $CH_3$,
- $n'_1$, $n''_1$ et $n_4$ = 0, 1 ou 2,
- $n'_2$, $n''_2$, $n_3$, $n'_3$ et $n_5$ = 0 ou 1,
- $n'_1 + n''_1 + n'_2 + n''_2 + n_3 + n'_3 + n_4 + n_5 \leq 2$,
- i vaut 1 ou 2,
- $W_i$ est un groupe de liaison trivalent tel que :

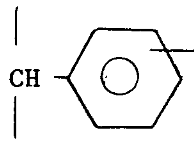

$(W_1)$

et/ou

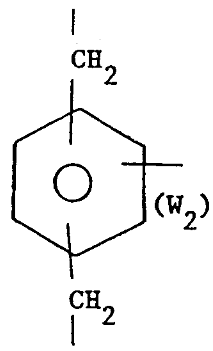

$(W_2)$

et dont les liaisons vers les groupes

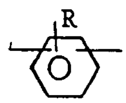

sont assurées par des atomes de carbone n'appartenant pas au groupement phényle de $W_i$, chacun des isomères B pouvant avoir des substituants $R'_i$, $R''_i$, $R_4$ à $R_{11}$ différents.

2. Compositions selon la revendication 1 caractérisées en ce que R, $R'_i$ et $R''_i$ sont des alkyles avec n compris entre 3 et 8, et de préférence des isopropyles.

3. Compositions selon les revendications 1 ou 2 caractérisées en ce que $R_1$ à $R_{11}$ sont des atomes d'hydrogène.

4. Compositions selon la revendications 1 caractérisées en ce que :
   - $R_1$ à $R_{11}$ sont des hydrogènes
   - R, $R'_i$ et $R''_i$ sont des isopropyles
   - i vaut 1, c'est-à-dire que le groupe de liaison $W_i$ est :

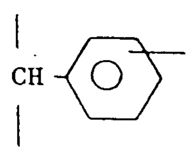

5. Compositions selon la revendication 4 caractérisées en ce que le mélange d'oligomères se trouve dans les proportions pondérales de :

   Composé A en mélange d'isomères :

   $n_1 + n_2 = 0$ compris entre 56 et 90 %
   $n_1 + n_2 = 1$ compris entre 7 et 28 %
   $n_1 + n_2 = 2$ compris entre 1,5 et 8 %
   $n_1 + n_2 = 3$ compris entre 0,1 et 3 %

   Composé B en mélange d'isomères :

   $n'_1 + n''_1 + n'_2 + n''_2 + n_3 + n'_3 + n_4 + n_5 = o$ compris entre 1,1 et 5%
   $n'_1 + n''_1 + n'_2 + n''_2 + n_3 + n'_3 + n_4 + n_5 = 1$ compris entre 0,25 et 1,5 %

$n'_1 + n''_1 + n'_2 + n''_2 + n_3 + n'_3 + n_4 + n_5 = 2$ compris entre 0,05 et 0,5 %

**6.** Procédé de fabrication des compositions d'oligomères de polyarylalcanes selon la revendication 1 caractérisé en ce que dans une première étape on fait réagir le chlore sur le toluène ou le xylène ou un mélange de toluène et de xylène en présence de générateur de radicaux, puis on élimine le toluène ou le xylène non réagi ou le toluène et le xyène non réagis, et dans une seconde étape on soumet le produit de réaction de cette première étape à l'action d'un halogénure minéral ou d'un acide minéral en présence d'un produit benzenique de formule :

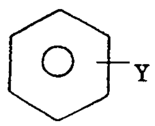 (III)

dans laquelle Y est une chaîne hydrocarbonée linéaire ou ramifiée ayant de 2 à 16 atomes de carbone ou un mélange de tels produits benzeniques.

**7.** Procédé selon la revendication 6 caractérisé en ce que dans la première étape on ne transforme en dérivé chloré que 10 à 30 % molaire du toluène ou du xylène ou du mélange de toluène et xylène engagé.

**8.** Procédé selon les revendications 6 ou 7, caractérisé en ce que dans la prémière étape on fait réagir le chlore sur le toluène.

**9.** Produit selon l'une des revendications 6 à 8, caractérisé en ce que le produit de formule (III) est un alkylbenzène, Y ayant de 3 à 8 atomes de carbone, ou un mélange de tels alkylbenzènes.

**10.** Procédé selon l'une des revendications 6 à 9, caractérisé en ce que le produit de formule (III) est

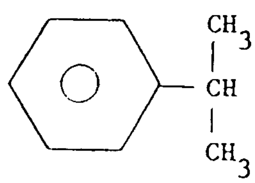

(l'isopropylbenzène ou cumène).

**11.** Procédé selon l'une des revendications 6 à 10, caractérisé en ce qu'on procède à une évaporation flash sur le produit obtenu en seconde étape.

## Claims

**1.** Polyarylalkane oligomer compositions consisting of the mixtures of two oligomers A and B, which are characterised in that:

- the oligomer A is a mixture of isomers of formula:

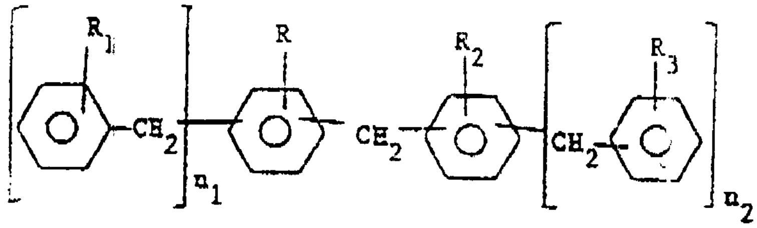

in which

R is a linear or branched hydrocarbon chain containing n carbon atoms, and n is between 2 and 16

$R_1$, $R_2$ and $R_3$ are identical or different and denote H or $CH_3$

$n_1$ and $n_2$ = 0, 1 and 2 with $n_1 + n_2 \leq 3$

it being possible for each of the isomers A to have different substituents R, $R_1$, $R_2$ and $R_3$:

- the oligomer B is a mixture of isomers of formula:

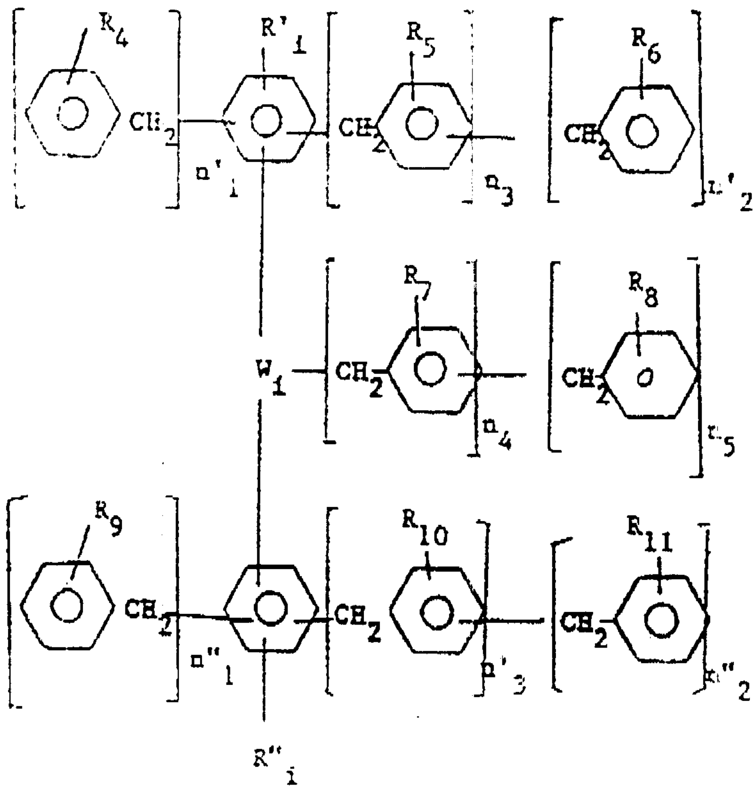

in which:

$R'_i$ and $R''_i$ are identical or different and have the same meaning as R,

$R_4$, $R_5$, $R_6$, $R_7$, $R_8$, $R_9$, $R_{10}$ and $R_{11}$ are identical or different and denote H or $CH_3$,

$n'_1$, $n''_1$ and $n_4$ = 0, 1 or 2,

$n'_2$, $n''_2$, $n_3$, $n'_3$ and $n_5$ - 0 or 1,

$n'_1 + n''_1 + n'_2 + n''_2 + n_3 + n'_3 + n_4 + n_5 \leq 2$

i has the value of 1 or 2,

$W_i$ is a trivalent connecting group such as:

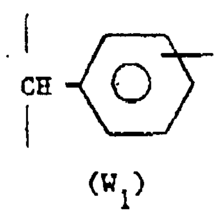

and/or

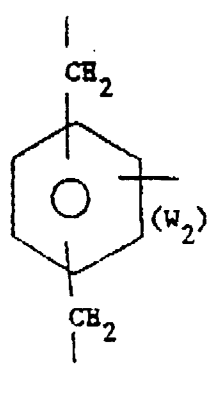

and in which the connections towards the groups

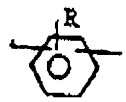

are provided by carbon atoms not forming part of the phenyl group of $W_i$, it being possible for each of the isomers B to have different substituents $R'_i$, $R''_i$, and $R_4$ to $R_{11}$.

2. Compositions according to Claim 1, characterised in that R, $R'_i$, and $R''_i$ are alkyls with n between 3 and 8, and preferably isopropyls.

3. Compositions according to Claims 1 or 2, characterised in that $R_1$ to $R_{11}$ are hydrogen atoms.

4. Compositions according to Claim 1, characterised in that:
   - $R_1$ to $R_{11}$ are hydrogens
   - R, $R'_i$ and $R''_i$ are isopropyls and
   - i has the value of 1, that is to say that the connecting group $W_i$ is:

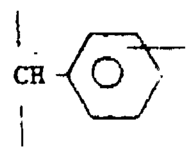

5. Compositions according to Claim 4, characterised in that the mixture of oligomers is in the proportions by weight of:

   Compound A as a mixture of isomers:

   $n_1 + n_2 = 0$ between 56 and 90%
   $n_1 + n_2 = 1$ between 7 and 28%
   $n_1 + n_2 = 2$ between 1.5 and 8%
   $n_1 + n_2 = 3$ between 0.1 and 3%

   Compound B as a mixture of isomers:

   $n'_1 + n''_1 + n'_2 + n''_2 + n_3 + n_3 + n_4 + n_5 = 0$ between 1.1 and 5%
   $n'_1 + n''_1 + n'_2 + n''_2 + n_3 + n'_3 + n_4 + n_5 = 1$ between 0.25 and 1.5%
   $n'_1 + n''_1 + n'_2 + n''_2 + n_3 + n'_3 + n_4 + n_5 = 2$ between 0.05 and 0.5%

6. Process for the manufacture of polyarylalkane oligomer compositions according to Claim 1, characterised in that, in a first stage, chlorine is reacted with toluene or xylene or a mixture of toluene and xylene in the presence of a radical generator, and then the unreacted toluene or xylene or the unreacted toluene and xylene are removed and, in a second stage, the reaction product from this first stage is subjected to the action of an inorganic halide or of an inorganic acid in the presence of a benzene-related product of formula:

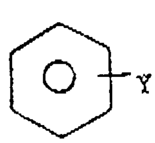

(III)

in which Y is a linear or branched hydrocarbon chain containing from 2 to 16 carbon atoms or a mixture

of such benzene-related products.

**7.** Process according to Claim 6, characterised in that, in the first stage, only 10 to 30 mol% of the toluene or xylene or of the mixture of toluene and xylene which is employed is converted into a chlorine derivative.

**8.** Process according to Claim 6 or 7, characterised in that, in the first stage, chlorine is reacted with toluene.

**9.** Process according to one of Claims 6 to 8, characterised in that the product of formula (III) is an alkylbenzene, Y containing from 3 to 8 carbon atoms, or a mixture of such alkylbenzenes.

**10.** Process according to one of Claims 6 to 9, characterised in that the product of formula (III) is

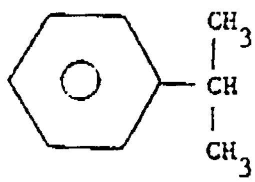

(isopropylbenzene or cumene).

**11.** Process according to one of Claims 6 to 10, characterized in that a flash evaporation is carried out on the product obtained in the second stage.

**Patentansprüche**

**1.** Zusammensetzungen von oligomeren Polyarylalkanen, bestehend aus einer Mischung von zwei Oligomeren A und B, dadurch gekennzeichnet, daß

- das Oligomere A eine Mischung von Isomeren der Formel

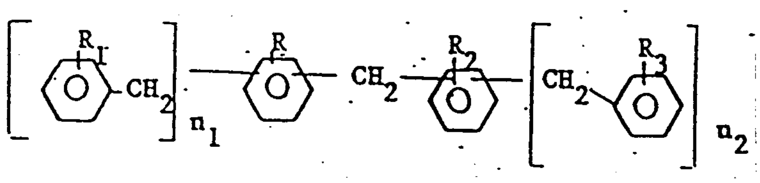

ist, worin

- R eine geradkettige oder verzweigte Kohlenwasserstoffkette mit n Kohlenstoffatomen ist und n zwischen 2 und 16 beträgt,
- $R_1$, $R_2$ und $R_3$ identisch oder verschieden sind und H oder $CH_3$ bedeuten,
- $n_1$ und $n_2$ = 0, 1 und 2 mit $n_1 + n_2 \leqq 3$ sind,
- jedes der Isomeren A verschiedene Substituenten R, $R_1$, $R_2$ und $R_3$ haben kann,

- das Oligomere B eine Mischung von Isomeren der Formel

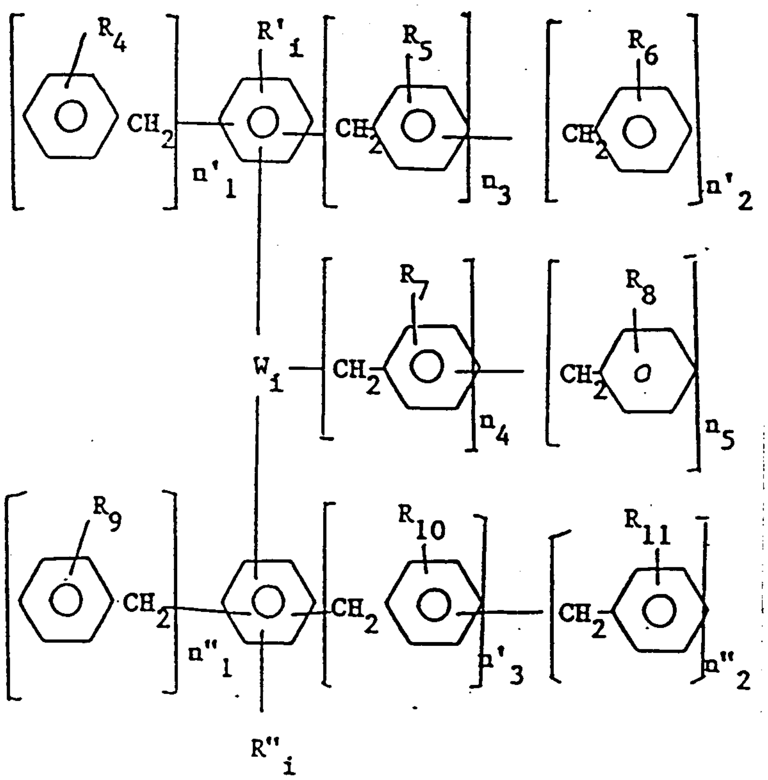

ist, worin

- $R'_i$ und $R''_i$ identisch oder verschieden sind und die gleiche Bedeutung wie R haben,
- $R_4$, $R_5$, $R_6$, $R_7$, $R_8$, $R_9$, $R_{10}$, $R_{11}$ gleich oder verschieden sind und H oder $CH_3$ bedeuten,
- $n'_1$, $n''_1$ und $n_4$ = 0, 1 oder 2,
- $n'_2$, $n''_2$, $n_3$ $n'_3$ und $n_5$ = 0 oder 1,
- $n'_1 + n''_1 + n'_2 + n''_2 + n_3 + n'_3 + n_4 + n_5 \leqq 2$ sind,
- i 1 oder 2 bedeutet,
- $W_i$ eine dreiwertige Verbindungsgruppe ist, wie

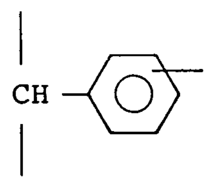

und/oder

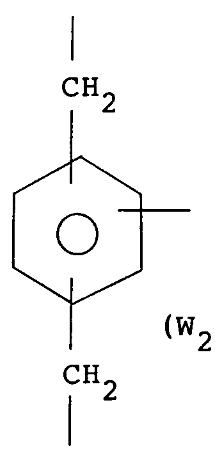

($W_1$) ($W_2$)

deren Bindungen zu den Gruppen

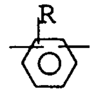

durch Kohlenstoffatome gesichert sind, die nicht zu der Phenylgruppe von $W_i$ gehören, wobei jedes der Isomeren B verschiedene Substituenten $R'_i$, $R''_i$, $R_4$ bis $R_{11}$ haben kann.

**2.** Zusammensetzungen nach Anspruch 1, dadurch gekennzeichnet, daß R, $R'_i$ und $R''_i$ Alkyle mit n zwischen 3 und 8 und vorzugsweise Isopropyle sind.

**3.** Zusammensetzungen nach den Ansprüchen 1 oder 2, dadurch gekennzeichnet, daß $R_1$ bis $R_{11}$ Wasserstoffatome sind.

**4.** Zusammensetzungen nach Anspruch 1, dadurch gekennzeichnet, daß
- $R_1$ bis $R_{11}$ Wasserstoffe sind,
- R, $R'_i$ und $R''_i$ Isopropyle sind,
- i 1 bedeutet, also die Verbindungsgruppe $W_i$

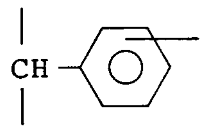

ist.

**5.** Zusammensetzungen nach Anspruch 4, dadurch gekennzeichnet, daß die Oligomerenmischung mit folgenden Gewichtsverhältnissen vorliegt:
Verbindung A als Mischung von Isomeren:
$n_1 + n_2 = 0$ beträgt zwischen 56 und 90%,
$n_1 + n_2 = 1$ beträgt zwischen 7 und 28 %,
$n_1 + n_2 = 2$ beträgt zwischen 1,5 und 8 %,
$n_1 + n_2 = 3$ beträgt zwischen 0,1 und 3 %,
Verbindung B als Mischung von Isomeren:
$n'_1 + n''_1 + n'_2 + n''_2 + n_3 + n'_3 + n_4 + n_5 = 0$ beträgt zwischen 1,1 und 5 %,
$n'_1 + n''_1 + n'_2 + n''_2 + n_3 + n'_3 + n_4 + n_5 = 1$ beträgt zwischen 0,25 und 1,5 %,
$n'_1 + n''_1 + n'_2 + n''_2 + n_3 + n'_3 + n_4 + n_5 = 2$ beträgt zwischen 0,05 und 0,5 %.

**6.** Verfahren zur Herstellung von Zusammensetzungen von Polyarylalkanoligomeren nach Anspruch 1, dadurch gekennzeichnet, daß man in einer ersten Stufe Chlor mit Toluol oder Xylol oder einer Mischung von Toluol und Xylol in Gegenwart eines Radikalbildners reagieren läßt, dann nicht umgesetztes Toluol oder Xylol oder nicht umgesetztes Toluol und Xylol entfernt und anschliessend in einer zweiten Stufe das Reaktionsprodukt dieser ersten Stufe der Einwirkung eines Mineralhalogenids oder einer Mineralsäure in Gegenwart einer benzolischen Verbindung der Formel

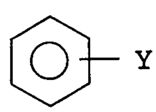 (III)

unterzieht, in der Y eine geradkettige oder verzweigte Kohlenwasserstoffkette mit 2 bis 16 Kohlenstoffatomen oder eine Mischung solcher benzolischen Verbindungen ist.

**7.** Verfahren nach Anspruch 6, dadurch gekennzeichnet, daß man in der ersten Stufe nur 10 bis 30 Mol% des eingesetzten Toluols oder Xylols oder der eingesetzten Mischung von Toluol und Xylol in das chlorierte Derivat umwandelt.

**8.** Verfahren nach den Ansprüchen 6 oder 7, dadurch gekennzeichnet, daß man in der ersten Stufe Chlor mit Toluol reagieren läßt.

**9.** Verfahren nach einem der Ansprüche 6 bis 8, dadurch gekennzeichnet, daß die Verbindung der Formel (III) ein Alkylbenzol, wobei Y 3 bis 8 Kohlenstoffatome aufweist, oder eine Mischung solcher Alkylbenzole ist.

**10.** Verfahren nach einem der Ansprüche 6 bis 9, dadurch gekennzeichnet, daß die Verbindung der Formel (III)

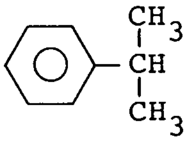

(Isopropylbenzol oder Cumol) ist.

**11.** Verfahren nach einem der Ansprüche 6 bis 10, dadurch gekennzeichnet,daß man das in der zweiten Stufe erhaltene Produkt einer Verdampfung durch Entspannung unterzieht.